# FASCICULE DE BREVET EUROPEEN

(11) **EP 1 967 268 B1**
(45) Date de publication et mention de la délivrance du brevet: **01.01.2014**
(21) Numéro de dépôt: 08300044.8
(22) Date de dépôt: 22.01.2008
(51) Int. Cl.: B01L 3/08, G01N 25/08, G01N 25/10, G01N 25/14, G01N 33/28

(54) **Collerette en verre solidaire de la branche latérale de la colonne du ballon de distillation**
Glasflansch verbunden mit dem Seitenarm der Säule des Destillierkolbens
Glass flange solidly attached to the lateral branch of the distillation flask column

(30) Priorité: 13.02.2007 FR 0753229
(43) Date de publication de la demande: 10.09.2008
(73) Titulaire: Instrumentation Scientifique de Laboratoire ISL, 14790 Verson (FR)
(72) Inventeur: Marie, Patrick, 14980 Rots (FR)
(74) Mandataire: Livet, Marie-José

(56) Documents cités:
- DE-U1-202004 008 920
- FR-A- 926 920
- FR-A- 2 178 755
- FR-A1- 2 815 413
- GB-A- 860 133
- US-A- 4 569 724
- US-A- 4 826 575

## Description

La présente invention a pour objet un procédé de branchement de la branche latérale d'un ballon de distillation mono matière sur un appareil de distillation, notamment sur un appareil de distillation automatique sous pression atmosphérique d'échantillons liquides, en particulier d'échantillons de produits pétroliers pour permettre d'effectuer la mesure des paramètres de distillation de ces échantillons en respectant des normes.

Un tel ballon de distillation est en règle générale en verre.

Il est connu que les caractéristiques de distillation des produits pétroliers sont représentatives des performances de ces produits ainsi que des risques qu'ils peuvent faire encourir à leurs utilisateurs.

La détermination de ces caractéristiques présente notamment une grande importance dans le cas de carburants destinés à l'industrie automobile ou à l'aviation où les problèmes liés à la sécurité sont primordiaux.

Ces caractéristiques sont en particulier des tables ou des courbes représentant le pourcentage d'un échantillon évaporé selon la température pendant une distillation ou encore le volume du résidu et les pertes.

Les spécialistes peuvent déduire de ces caractéristiques quel sera le comportement d'un produit pétrolier donné dans une situation donnée et donc déterminer si ce produit peut ou non être utilisé en toute sécurité, ce de manière à permettre d'obtenir des performances recherchées.

Dans ce contexte, les spécialistes ont édicté différentes normes qui définissent très précisément les conditions dans lesquelles doivent être obtenues de telles caractéristiques de distillation.

Par suite, pour donner des résultats exploitables, les distillations doivent être mises en oeuvre en respectant scrupuleusement ces normes.

Il existe actuellement sur le marché, différents appareils de distillation automatique permettant d'effectuer la mesure des paramètres de distillation d'un échantillon en respectant ces normes.

Ces appareils de distillation normalisés renferment en règle générale :
- un élément chauffant,
- un ballon de distillation se prolongeant par une colonne de distillation pouvant être fermée par un bouchon étanche muni d'un thermomètre permettant de mesurer la température des vapeurs évaporées et équipée d'une branche latérale destinée à être branchée sur un tube condenseur,
- un cylindre collecteur permettant de recueillir le condensât et équipé d'organes de mesure de la quantité de condensât ainsi recueillie en fonction du temps, et
- des moyens de commande et de régulation permettant de commander et de faire varier dans le temps une grandeur de fonctionnement de l'élément chauffant, notamment la température ou la puissance électrique de cet élément de manière à obtenir des caractéristiques et/ou des courbes de distillation conformément à une norme prédéfinie.

Lors de la mise en oeuvre d'une opération de distillation d'un échantillon, il est bien entendu essentiel que la branche latérale de la colonne de distillation soit reliée de façon rigoureusement étanche au tube condenseur de façon à éviter toute perte d'échantillon en cours de distillation.

Dans ce but, la branche latérale de la colonne de distillation des ballons de distillation actuellement utilisés est équipée sur sa périphérie externe, au niveau de son extrémité libre d'un embout annulaire en silicone fixé solidairement à celle-ci.

La branche latérale de la colonne du ballon ainsi équipée de cet embout en silicone, est emmanchée sur le tube condenseur.

Or, la mise en place de cet embout annulaire en silicone est particulièrement incommode.

La présente invention a pour objet de proposer un ballon de distillation permettant de remédier à cet inconvénient.

A cet effet, l'invention a pour objet un procédé de branchement de la branche latérale d'un ballon de distillation mono matière sur un appareil de distillation, notamment sur un appareil de distillation automatique sous pression atmosphérique d'échantillons liquides, en particulier d'échantillons de produits pétroliers pour permettre d'effectuer la mesure des paramètres de distillation de ces échantillons en respectant des normes, cet appareil de distillation comportant un élément chauffant et un tube condenseur relié à un cylindre collecteur permettant de recueillir le condensat.

Ce procédé est caractérisé par la succession des étapes suivantes :
- lors de la fabrication du ballon de distillation, on forme une collerette à la périphérie externe de la branche latérale de ce ballon, en deçà de son extrémité lire,
- on introduit l'extrémité libre de la branche latérale en silicone autour de l'extrémité libre du tube condenseur,
- on introduit l'extrémité libre de la branche latérale du ballon de distillation dans le manchon et dans le tube condensateur, et
- on emmanche à force la collerette dans le manchon de façon à permettre une liaison étanche du ballon de distillation et du tube condensateur.

La collerette est réalisée dans le même matériau que les autres parties du ballon de distillation en règle générale en verre ; on obtient donc ainsi un ballon de distillation mono matière.

Pour relier le ballon de distillation une fois rempli de l'échantillon à analyser au tube condenseur, il suffit d'emmancher à force, la collerette en verre de la branche latérale de sa colonne de distillation dans un manchon en silicone, monté à force et à demeure à l'extrémité libre du tube condenseur.

Les dimensions de ce manchon doivent bien entendu être choisies de manière à garantir l'étanchéité nécessaire après montage de ces éléments.

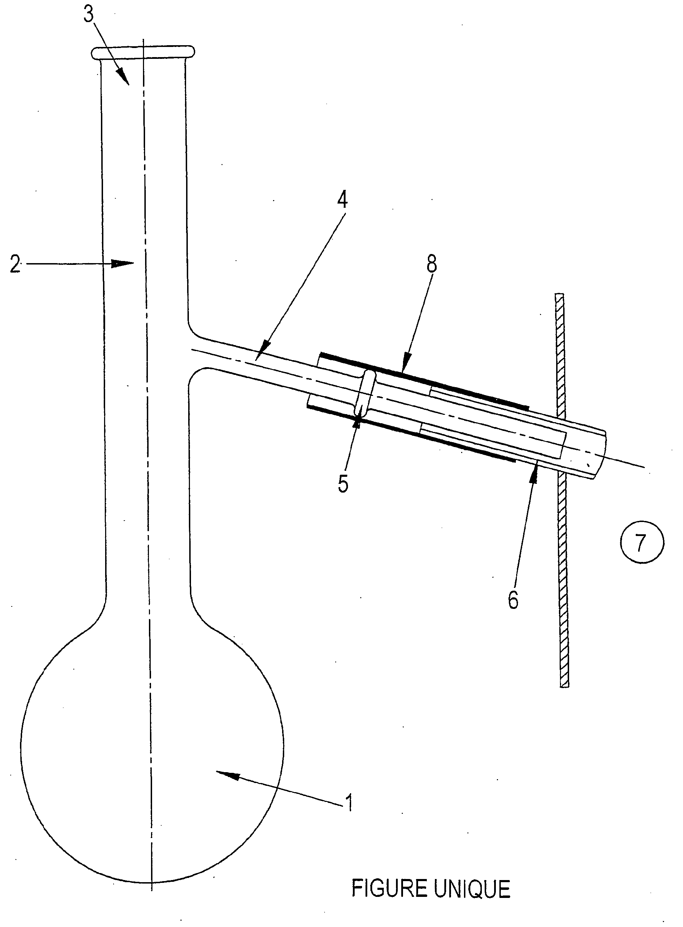

Les caractéristiques du procédé qui fait l'objet de l'invention seront décrites plus en détail en se référant au dessin non limitatif annexé qui est une vue en coupe d'un ballon de distillation monté à l'extrémité du tube condenseur d'un appareil de distillation.

Selon la figure, le ballon de distillation 1 qui est réalisé en verre se prolonge par une colonne de distillation 2 pouvant être fermée à son extrémité libre 3 par un bouchon muni d'un thermomètre non représenté et équipée à sa partie médiane d'une branche latérale 4.

La branche latérale 4 est munie d'une collerette 5 à sa périphérie externe.

Cette collerette 5 permet de relier de façon étanche la branche latérale 4 de la colonne de distillation 2 à un tube condenseur 6 pénétrant dans une enceinte réfrigérée 7 pour condenser les vapeurs évaporées et recueillir le condensât dans un cylindre collecteur non représenté.

A cet effet, un manchon en silicone 8 est monté à demeure autour de l'extrémité du tube condenseur 6.

L'extrémité libre de la branche latérale 4 est introduite dans ce manchon en silicone 8 et dans le tube condenseur 6 et maintenue à force dans ce manchon grâce à la présence de la collerette 5.

L'étanchéité entre la branche latérale 4 et le tube condenseur 6 est ainsi garantie.

## Revendications

1. Procédé de branchement de la branche latérale (4) d'un ballon de distillation mono matière (1) sur un appareil de distillation, notamment sur un appareil de distillation automatique sous pression atmosphérique d'échantillons liquides, en particulier d'échantillons de produits pétroliers pour permettre d'effectuer la mesure des paramètres de distillation de ces échantillons en respectant des normes, cet appareil de distillation comportant un élément chauffant et un tube condenseur (6) relié à un cylindre collecteur permettant de recueillir le condensat,
**caractérisé par** la succession des étapes suivantes :
- lors de la fabrication du ballon de distillation (1), on forme une collerette (5) à la périphérie externe de la branche latérale (4) de ce ballon, en deçà de son extrémité libre,
- on monte à force et à demeure un manchon (8), en silicone autour de l'extrémité libre du tube condenseur (6),
- on introduit l'extrémité libre de la branche latérale (4) du ballon de distillation (1) dans le manchon (8) et dans le tube condensateur (6), et
- on emmanche à force la collerette (5) dans le manchon (8) de façon à permettre une liaison étanche du ballon de distillation (1) et du tube condensateur (6).

## Patentansprüche

1. Verfahren zum Anschluss des Seitenarms (4) eines aus einem einzigen Material hergestellten Destillierkolbens (1) an einen Apparat zum Destillieren, insbesondere an einen Apparat zum automatischen Destillieren von flüssigen Proben unter Atmosphärendruck, insbesondere von Ölproduktproben, um das Messen der Destillierparameter der Proben im Hinblick auf Normen zu ermöglichen, wobei der Apparat zum Destillieren ein Heizelement und eine mit einem Sammelzylinder verbundene Kondensierröhre (6), die das Kondensat sammeln kann, aufweist, **gekennzeichnet durch** die Folge der nachstehenden Schritte:
- bei der Herstellung des Destillierkolbens (1) wird ein Flansch (5) am Außenumfang des Seitenarms (4) des Kolbens, diesseits seines freien Endes, gebildet,
- es wird unter Kraft und dauerhaft eine Muffe aus Silikon (8) um das freie Ende der Kondensierröhre (6) herum montiert,
- es wird das freie Ende des Seitenarms (4) des Destillierkolbens (1) in die Muffe (8) und in die Kondensierröhre (6) eingeführt, und
- es wird unter Kraft der Flansch (5) in die Muffe (8) derart eingeführt, dass eine dichte Verbindung zwischen dem Destillierkolben (1) und der Kondensierröhre (6) ermöglicht wird.

## Claims

1. Process for connection of the side arm (4) of a single-material distillation flask (1) to distillation equipment, in particular equipment for the automatic distillation of liquid samples under atmospheric pressure, in particular samples of petroleum products so that the distillation parameters of these samples may be measured in compliance with standards, this distillation equipment comprising a heating element and a condenser tube (6) connected to a collecting cylinder for recovery of the condensate,
**characterised by** the following sequence of stages:
- when manufacturing the distillation flask (1), a collar (5) is formed on the outer periphery of the side arm (4) of that flask, short of its free extremity,
- a silicone sleeve (8) is permanently force-fitted around the free end of the condenser tube (6),
- the free end of the side arm (4) of the distillation flask (1) is inserted into the sleeve (8) and the condenser tube (6), and
- the collar (5) is force-fitted into the sleeve (8) in such a way as to form a leaktight connection between the distillation flask (1) and the condenser tube (6).
